# EUROPÄISCHE PATENTSCHRIFT

(11) **EP 1 438 090 B1**
(45) Veröffentlichungstag und Bekanntmachung des Hinweises auf die Patenterteilung: **04.03.2009**
(21) Anmeldenummer: 02762365.1
(22) Anmeldetag: 16.07.2002
(51) Int. Cl.: A61M 1/16

(54) **BEHÄLTNIS ZUR VERWENDUNG IN DER DIALYSE**
CONTAINER FOR USING FOR DIALYSIS
CONTENANT DESTINE A ETRE UTILISE EN DIALYSE

(30) Priorität: 23.10.2001 DE 10152105
(43) Veröffentlichungstag der Anmeldung: 21.07.2004
(60) Teilanmeldung: 08020073.6
(73) Patentinhaber: Fresenius Medical Care Deutschland GmbH, 61352 Bad Homburg (DE)
(72) Erfinder: DUMON D'AYOT, Francois, F-69005 Lyon (FR); DUPIN, Thierry, F-69690 Besseway (FR); LAFFAY, Philippe, F-69110 Fou Les Lyon (FR); GRAF, Thomas, F-69380 H. Jean des Vigues (FR)
(74) Vertreter: Laufhütte, Dieter
(86) Internationale Anmeldenummer: PCT/EP2002/007922
(87) Internationale Veröffentlichungsnummer: WO 2003/035146

(56) Entgegenhaltungen:
- EP-A- 0 475 825
- EP-A- 0 484 751
- EP-A- 0 665 026
- FR-A- 2 766 797
- US-A- 5 385 564
- US-A- 5 387 237

## Beschreibung

Die Erfindung betrifft ein Behältnis zur Verwendung in der Dialyse nach dem Oberbegriff des Anspruchs 1.

Bei Dialysesystemen ist es bisher verbreitet, das für den Dialysevorgang notwendige Dialysat erst unmittelbar vor der Dialysesitzung herzustellen. Hierzu wird in einem Behältnis das Dialysatkonzentrat in fester Form vorgelegt, wobei dieses üblicherweise in Form von Pulvern, Granulaten oder Pastillen, d.h. Presslingen aus Pulver, vorgelegt wird. Zur Bildung des Dialysats muß das feste Dialysatkonzentrat in Wasser gelöst werden. Hierzu wird das Behältnis mit dem festen Dialysatkonzentrat in die Dialysiervorrichtung eingespannt und üblicherweise wird das Wasser, in welchem das Dialysatkonzentrat aufgelöst werden soll, auf einer Seite in das Behältnis eingeleitet und auf der gegenüberliegenden Seite wieder abgezogen. Hierbei ergibt sich die Problematik, daß die abgezogene Lösung nicht gesättigt ist, so daß die Zusammensetzung.des Dialysats variiert,

Aus der EP 0 475 825 A1 ist bereits ein Behältnis mit den Merkmalen des Oberbegriffs des Anspruchs 1 bekannt. Hier erfolgt allerdings der Zulauf des Wassers über eine gesonderte Leitung, die neben dem Behältnis, in welchem das Granulat bzw. Pulver vorgelegt ist, verläuft.

Aufgabe der vorliegenden Erfindung ist es, ein Behältnis an die Hand zu geben, bei dem sichergestellt ist, daß auch bei schwerer löslichen Komponenten die zur Dialysesitzung bereitgestellte Lösung gesättigt ist.

Erfingdungsgemäß wird diese Aufgabe ausgehend von einem bekannten Behältnis dadurch gelöst, daß sowohl der Zulauf wie auch der Ablauf auf einer Seite des Behältnisses, vorzugsweise im Bodenbereich, angeordnet sind. Wesentlich ist es hier, daß sowohl Zulauf und Ablauf in Nachbarschaft des als Feststoff vorgelegten Dialysatkonzentrats liegen. Des weiteren weist das Behältnis in seinem Inneren eine Trennwand zur Bildung verschiedener symmetrischer Bereiche im Behältnis auf, wobei die Trennwand aber zumindest soweit durchbrochen ist, dass ein Fluidaustausch zwischen den einzelnen Bereichen möglich ist, so dass im Betrieb Wasser durch das Salzkonzentrat geführt werden kann. Hierdurch wird sichergestellt, daß das frische Wasser die in fester Form vorliegenden Dialysatkonzentratteilchen umspült und aufgrund der dadurch bedingten turbulenten Strömung und Wirbelbildung zu einer schnellstmöglichen Lösung beiträgt. Die Anordnung des Auslaufs auf der gleichen Seite stellt sicher, daß die Flüssigkeit auch bei einem kontinuierlichen Betrieb eine möglichst lange Verweilzeit im noch nicht aufgelösten Dialysatkonzentrat hat, so daß hier eine Sättigung der Lösung auch bei schlecht lösenden Salzen erreicht werden kann. Aufgrund dieser Anordnung können auch die Dichteunterschiede ausgeglichen werden.

Aus dem US-Patent Nr. 5,385,564 ist bereits eine Lösung bekannt, in welcher ebenfalls in einem Behältnis die granulatförmige Dialysatkonzentration vorgelegt ist und bei der das Wasser über einen Anschluß in das Behältnis eingeleitet wird. Allerdings muß zunächst das Wasser vollständig in das Behältnis geleitet werden und nach entsprechendem Lösen des Konzentrats in dem Wasser wird das fertige Dialysat wieder durch die gleiche Öffnung im Behältnis ausgeleitet. Mit dieser Lösung ist ein kontinuierlicher Betrieb nicht möglich.

Bevorzugte Ausgestaltungen der Erfindung ergeben sich aus den sich an den Hauptanspruch anschließenden Unteransprüchen.

Demnach kann das Behältnis vorteilhaft in Form eines Beutels ausgebildet sein, der aus zwei zusammengeschweißten Folien gebildet ist, in dessen Bodenbereich der Zulauf bzw. Ablauf gebildet wird, wobei Zulauf und Ablauf vorzugsweise in Form von Konnektoren ausgebildet sind, die mit der Dialysiervorrichtung verbindbar sind.

Besonders vorteilhaft sind sowohl im Zulauf wie auch im Ablauf jeweils Filter mit einer Porosität von 50 bis 500 µm angeordnet.

Vorteilhaft kann die Trennwand bei diesem Behältnis besonders einfach hergestellt werden, wenn das Behältnis aus einem Beutel besteht, in dem die Seitenwände des Beutels an den entsprechenden Stellen, an denen die Trennwand gebildet sein soll, miteinander verschweißt werden.

Besonders vorteilhaft läßt sich das Behältnis einsetzen, wenn das Dialysatkonzentrat unter anderem Bicarbonat oder Natriumchlorid enthält.

Weitere Einzelheiten und Vorteile der Erfindung werden anhand eines in der Zeichnung dargestellten Ausführungsbeispieles näher erläutert. Es zeigen:
- Fig. 1:: eine schematische Darstellung einer nicht zur Erfindung gehören- den Ausführungsvariante,
- Fig. 2, 2a:: eine graphische Verdeutlichung des Einleitens von Wasser in ei- nem Beutel gemäß Figur 1, teilweise vergrößert,
- Fig. 3, 5, 7, 8:: unterschiedliche Ausführungsvarianten in vereinfachter Schnittdar- stellung, die ebenfalls nicht zur Erfindung gehören,
- Fig. 4, 6: Ausführungsvarianten der vorliegenden Erfindung in vereinfachter Schnittdarstellung und
- Fig. 9:: ein Diagramm, in welchem die Flüssigkeit in Abhängigkeit der Temperatur über die Zeit dargestellt ist.

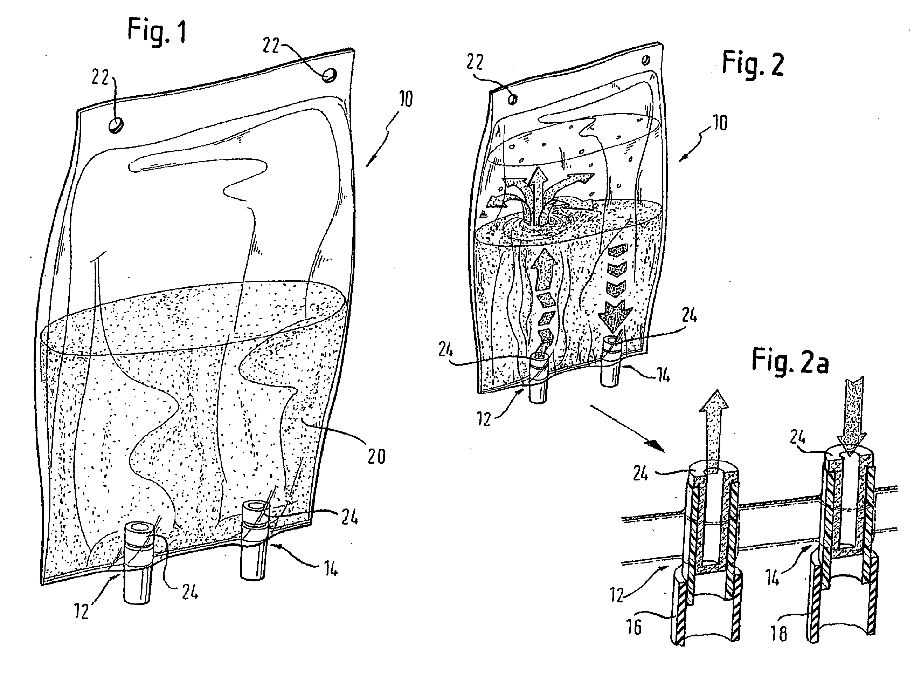

In der Ausführungsvariante gemäß Figur 1 besteht das Behältnis 10 aus einem Beutel, der aus zwei Kunststoffolien gefertigt ist, die seitlich miteinander verschweißt sind. Im Bodenbereich des Beutels 10 sind ein Einlaß 12 und ein Auslaß 14 vorgesehen, wobei diese im hier dargestellten Ausführungsbeispiel als rohrförmige Ansätze ausgebildet sind, auf die entsprechende Schlauchenden 16, 18 (vgl. Fig. 2a) aufschiebbar sind. Im unteren Teil des Beutels ist das aus Pulver, Granulat oder Pastillen bzw. Gemischen hiervon bestehende Dialysatkonzentrat 20 angeordnet. Im oberen Bereich weist der Beutel 10 zwei Aufnahmeöffnungen 22 auf, mittels derer dieser Beutel aufgehängt werden kann. Im Zulauf 12 bzw. Ablauf 14 sind jeweils in das Röhrchen einsteckbare Filter 24 mit einer Porosität von 50 bis 500 µm angeordnet. Im Schnitt sind diese Filter 24 ebenfalls in Figur 2a dargestellt.

In Figur 2 ist der Strömungsverlauf des durch den Zulauf 12 einströmenden Wassers gezeigt. Insbesondere ist hier durch die Pfeile angedeutet, daß entlang eines vergleichsweise breiteren Kanals das Dialysatkonzentrat durchströmt und dadurch wie auch durch die Dichteunterschiede zwischen konzentrierter und wenig konzentrierter Lösung auch verwirbelt wird. Von dem oberen Bereich des Beutels muß das Wasser nochmals das gesamte Haufwerk aus dem Dialysatkonzentrat durchströmen, um dann durch den Ablauf 14 in gesättigter Form abgezogen zu werden. Die Filter 24 verhindern jeweils ein Durchtreten von noch nicht aufgelöstem Dialysatkonzentrat in die Schlauchleitungen 16 bzw. 18.

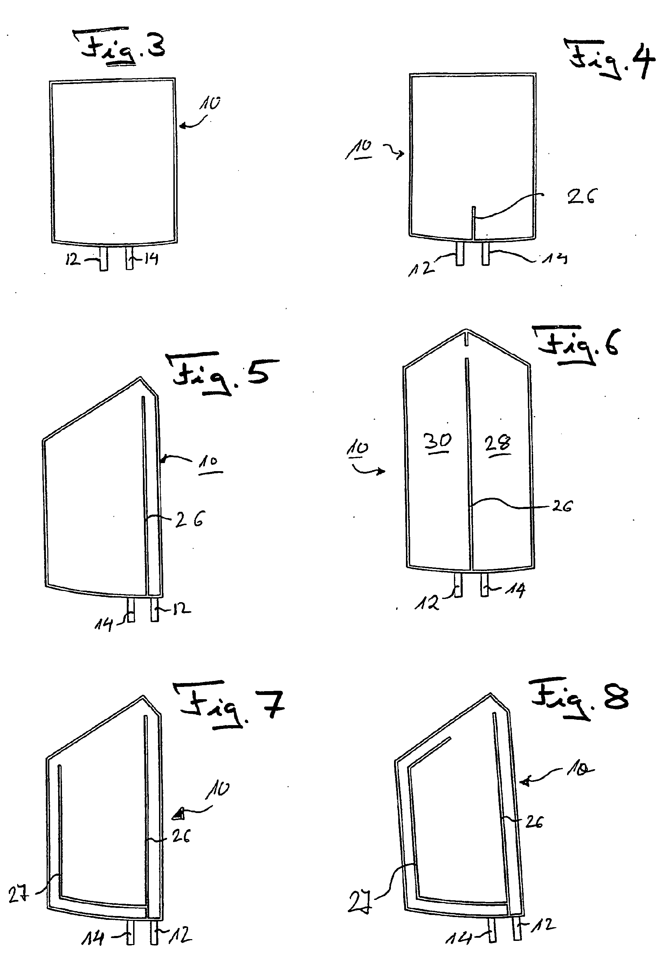

Anhand der Figuren 3 bis 8 können unterschiedliche Ausführungsvarianten des erfindungsgemäßen Behältnisses 10, das hier jeweils Beutelform aufweist, erläutert werden. Dabei ist die Beutelform eine einfache Ausführungsform, die aber nicht zwingend gemäß der vorliegenden Erfindung vorgeschrieben ist.

Die Figur 3, die eine nicht im Rahmen der Erfindung liegende Ausführung darstellt, zeigt einen Schnitt, der der Ausführungsform gemäß der Figuren 1 und 2 entspricht. Von dieser einfachsten Ausführungsvariante unterscheiden sich die Figuren 4 bis 8 dahingehend, daß hier im Inneren des Beutels jeweils Trennwände 26 vorgesehen sind, die hier unterschiedliche Bereiche im Beutel bilden. Hierdurch wird ein labyrinthartiges Hindurchtreten des frischen Wassers durch das Haufwerk des Dialysatkonzentrats erreicht.

In der eine erfinderische Ausführung zeigende Figur 4 ist die Trennwand 26 zwischen dem Zulauf 12 und dem Ablauf 14 angeordnet und dient hier im wesentlichen dazu, eine Kurzschlußströmung zwischen Zulauf und Ablauf zu verhindern. In oder Ausführungsvariante gemäß Figur 5 (die nicht zur Erfindung gehört) sind zwei Teilbereiche innerhalb des Beutels geschaffen, die eine sehr unterschiedliche Größe aufweisen. Hier sind zwar Zulauf und Ablauf auch im unteren Bereich des Beutels gezeigt. Grundsätzlich könnte diese aber auch um 180° gedreht werden. In diesem Fall wäre in dem schmaleren Bereich der Zulauf anzuordnen. Durch die Ausbildung der Trennwand wird hier sichergestellt, daß das frische Wasser in den Bereich des feststofförmigen Dialysatkonzentrats eingeleitet wird und dieses durchläuft, bevor die gesättigte Lösung dann aus dem Auslauf 14 wieder ausgeleitet wird.

In der die erfinderische Ausführungsvariante zeigenden Figur 6 sind durch die Trennwand 26 zwei symmetrische Kammern 28 und 30 gebildet.

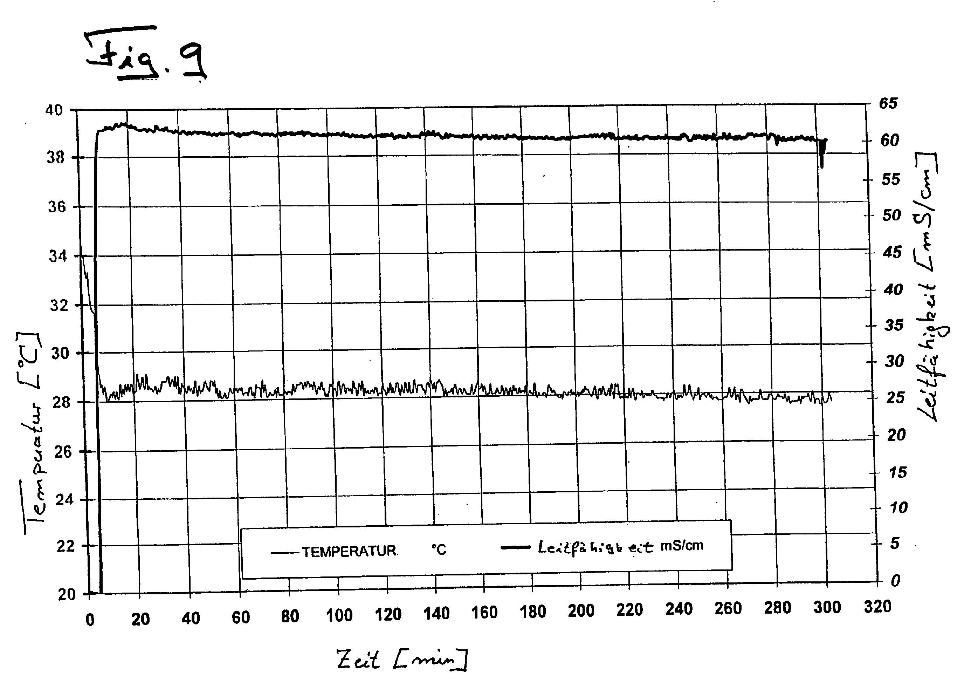

Die ebenfalls nicht im Rahmen der Erfindung liegende Figur 7 entspricht im wesentlichen derjenigen gemäß Figur 5 und führt zu einem ähnlich einsetzbaren Beutel. Hier ist lediglich noch durch eine zusätzliche Trennwand 27 innerhalb des Beutels eine langgestreckte Kammer gebildet, die den Zulauf zum Ablauf 14 definiert. Diese Kammer ist in Figur 8 entsprechend der Darstellung noch länger ausgeführt. Die Ausführungsvarianten gemäß der Figuren 5 bis 8 weisen jeweils Beutel auf, die zu einer Seite hin dreieckförmig geformt sind. Dies macht insbesondere dann Sinn, wenn die Beutel gegenüber der Darstellung hier um 180° gedreht sind, da sich dann im Bereich der Spitze des Dreiecks, also im untersten Bereich des Beutels das noch nicht gelöste Dialysatkonzentrat sammelt, wobei hier durch die entsprechende Ausbildung der Kammern durch die Trennwände sichergestellt ist, daß die Flüssigkeit diesen Bereich des Beutels auch durchströmt. In Figur 9 ist schließlich ein Diagramm gezeigt, aus dem hervorgeht, daß bei konstanter Temperatur bei der Herstellung des Dialysats eine konstante Sättigungskonzentration erreichbar ist. Die Sättigung der Lösung wird hier durch Leitfähigkeitsmessung ermittelt. Es ergibt sich hier bei einer konstanten Temperatur von etwas mehr als 28°C eine ebenfalls konstante Leitfähigkeit von ca. 60 mS/cm, wobei hier das Dialysat am Auslauf 14 in eine Anordnung gemäß der Figur 1 über den entsprechenden Zeitraum gemessen wurde.

## Patentansprüche

1. Behältnis (10) zur Verwendung in der Dialyse enthaltend eine bestimmte Menge eines Salzkonzentrats (20) in Form von Pulver, Granulat oder Pastillen oder Mischungen davon mit einem Zulauf (12) für das Wasser und mit einem an einem Dialysator anschließbaren Ablauf (14), wobei sowohl Zulauf (12) wie auch Ablauf (14) auf der Seite des Behältnisses (10) angeordnet sind, auf der sich das Salzkonzentrat (20) befindet, so dass sowohl Zulauf (12) wie auch Ablauf (14) in unmittelbarer Nachbarschaft des Salzkonzentrates (20) liegen,
**dadurch gekennzeichnet,**
**daß** es in seinem Inneren eine Trennwand (26) zur Bildung verschiedener symmetrischer Bereiche im Behältnis aufweist, wobei die Trennwand (26) aber zumindest soweit durchbrochen ist, daß ein Fluidaustausch zwischen den einzelnen Bereichen möglich ist, so dass im Betrieb Wasser durch das Salzkonzentrat geführt wird.

2. Behältnis nach Anspruch 1, **dadurch gekennzeichnet, daß** es in Form eines Beutels (10) ausgebildet ist, der aus zwei zusammengeschweißten Folien gebildet ist, in dessen Bodenbereich den Zulauf (12) bzw. den Ablauf (14) bildende Konnektoren angeordnet sind.

3. Behältnis nach Anspruch 1 oder 2, **dadurch gekennzeichnet, daß** im Zulauf (12) bzw. Ablauf (14) jeweils ein Filter (24) mit einer Porosität von 50 bis 500 µm angeordnet ist.

4. Behältnis nach Anspruch 1, **dadurch gekennzeichnet, daß** für den Fall, daß das Behältnis aus einem Beutel (10) besteht, die Trennwand (26) durch Verschweißen der die Seitenwände des Beutels bildenden Kunststoffolien gebildet ist.

5. Behältnis nach einem der Ansprüche 1 bis 4, **dadurch gekennzeichnet, daß** es unter anderem Bicarbonat oder Natriumchlorid enthält.

## Claims

1. A container (10) for use in dialysis containing a given amount of a salt concentrate (20) in the form of powder, granules or pastilles or mixtures thereof, having a feed (12) for the water and a discharge (14) connectable to a dialyser, wherein both the feed (12) and the discharge (14) are arranged on the side of the container (10) on which the salt concentrate (20) is disposed so that both the feed (12) and also the discharge (14) are in the immediate proximity of the salt concentrate (20),
**characterised in that**
in its interior it has a partition (26) for forming different symmetrical regions in the container, wherein however the partition (26) is apertured at least to such an extent that fluid exchange between the individual regions is possible so that in operation water is passed through the salt concentrate.

2. A container according to claim 1 **characterised in that** it is in the form of a bag (10) formed from two films which are welded together, connectors forming the feed (12) and the discharge (14) respectively being arranged in the bottom region of the bag.

3. A container according to claim 1 or claim 2 **characterised in that** a respective filter (24) of a porosity of 50 to 500 µm is arranged in the feed (12) and the discharge (14) respectively.

4. A container according to claim 1 **characterised in that**, for the situation where the container comprises a bag (10), the partition (26) is formed by welding the plastic films forming the side walls of the bag.

5. A container according to one of claims 1 to 4 **characterised in that** it contains inter alia bicarbonate or sodium chloride.

## Revendications

1. Contenant (10) pour utilisation en dialyse, contenant une quantité définie d'un concentré de sel (20) sous forme de poudre, granulées ou pastilles ou des mélanges de ceux-ci avec un afflux (12) pour l'eau et avec une évacuation (14) pouvant être raccordée à un dialyseur, où à la fois l'afflux (12) et aussi l'évacuation (14) sont disposés sur le côté du contenant (10), sur lequel se trouve le concentré de sel (20) de sorte qu'à la fois l'afflux (12) et aussi l'évacuation (14) se situent au voisinage direct du concentré de sel (20),
**caractérisé**
**en ce qu'**il présente dans son intérieur une paroi de séparation (26) pour la formation de différentes zones symétriques dans le contenant, où la paroi de séparation (26) est ajoutée au moins suffisamment pour qu'un échange de fluide entre les zones individuelles soit possible, de sorte qu'en cours de fonctionnement, l'eau est guidée à travers le concentré de sel.

2. Contenant selon la revendication 1, **caractérisé en ce qu'**il est réalisé sous forme d'un sachet (10) qui est formé par deux feuilles assemblées par soudage, dans la zone de fond duquel sont disposés des connecteurs formant l'afflux (12) respectivement l'évacuation (14).

3. Contenant selon la revendication 1 ou 2, **caractérisé en ce qu'**est disposé dans l'afflux (12) respectivement l'évacuation (14) respectivement un filtre (24) avec une porosité de 50 à 500 µm.

4. Contenant selon la revendication 1, **caractérisé en ce que** pour le cas où le contenant est constitué d'un sachet (10), la paroi de séparation (26) est formée par soudage des feuilles en matériau synthétique formant les parois latérales du sachet.

5. Contenant selon l'une des revendications 1 à 4, **caractérisé en ce qu'**il contient entre autres du bicarbonate ou du chlorure de sodium.
